# EUROPÄISCHE PATENTSCHRIFT

(11) **EP 2 097 367 B1**
(45) Veröffentlichungstag und Bekanntmachung des Hinweises auf die Patenterteilung: **30.11.2011**
(21) Anmeldenummer: 07822023.3
(22) Anmeldetag: 30.10.2007
(51) Int. Cl.: C07C 67/03, C07C 69/24, C07C 69/52, C07C 45/52, C07C 47/22, C11C 3/10, C12P 7/20, C12P 7/62

(54) **VERFAHREN ZUR HERSTELLUNG VON FETTSÄUREALKYLESTERN UND ACROLEIN AUS TRIGLYCERIDEN**
PROCESS FOR PREPARING ALKYL ESTERS OF FATTY ACIDS AND ACROLEIN FROM TRIGLYCERIDES
PROCÉDÉ DE FABRICATION D'ESTERS ALKYLIQUES D'ACIDES GRAS ET D'ACROLÉINE À PARTIR DE TRIGLYCÉRIDES

(30) Priorität: 20.11.2006 DE 102006054519
(43) Veröffentlichungstag der Anmeldung: 09.09.2009
(73) Patentinhaber: Evonik Degussa GmbH, 45128 Essen (DE)
(72) Erfinder: REDLINGSHÖFER, Hubert, 91481 Münchsteinach (DE); WECKBECKER, Christoph, 63584 Gründau-Lieblos (DE); HUTHMACHER, Klaus, 63571 Gelnhausen (DE)
(86) Internationale Anmeldenummer: PCT/EP2007/061669
(87) Internationale Veröffentlichungsnummer: WO 2008/061860

(56) Entgegenhaltungen:
- WO-A-2005/021697
- DE-C1- 4 238 493

## Beschreibung

Die Erfindung betrifft ein Verfahren zur Herstellung von Fettsäurealkylestern und Acrolein aus Triglyceriden unter Verwendung von geeigneten Katalysatoren.

Triglyeride können beispielsweise durch folgende Formel beschrieben werden: 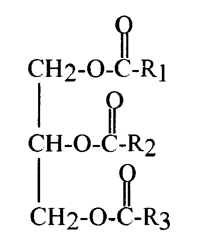 in der
R₁ bis R₃= C₁₀ bis C₃₀-Alkyl bedeuten.

Diese Triglyceride sind beispielsweise wichtiger Bestandteil von künstlichen und natürlichen Fetten oder Pflanzenölen, wie z.B. Palmöl, Sonnenblumenöl, Sojaöl oder Rapsöl, das in der Biodiesel-Produktion eingesetzt wird. Die erfindungsgemäß eingesetzten Triglyceride können in verunreinigter Form oder in Form von Mischungen vorliegen.

Fettsäurealkylester oder Biodiesel werden im vorliegenden Zusammenhang durch folgende Formel beschrieben: 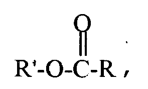 in der R = R₁ bis R₃ und R'= C₁ bis C₁₀-Alkyl oder C₃- bis C₆- Cycloalkyl bedeuten.

Gemische diese Fettsäurealkylester sind Hauptbestandteil des Biodiesel. Bevorzugt stellt R' eine CH₃- oder C₂H₅-Gruppe dar, meistens jedoch eine CH₃- Gruppe, da bei der Biodiesel-Produktion meist Methylate in methanolischer Lösung eingesetzt werden. Die Umesterung der Triglyceride kann auch mit anderen Alkoholen zu entsprechenden Fettsäurestern durchgeführt werden.

Einen Überblick über den Stand der Verwendung von Pflanzenölen und weiteren Fetten zur Herstellung von Biodiesel wird beschrieben in G. Knothe, J. Van Gerpen, J. Krahl, The Biodiesel Handbook, OACS Verlag, 2005.

Im allgemeinen kann die Umesterung von Triglyceriden zu Fettsäurealkylestern durch saure oder basische Katalyse beschleunigt werden. Technisch wird vorwiegend die schnellere homogene Basenkatalyse verwendet. Dabei werden bevorzugt Natrium- oder Kaliummethylate eingesetzt.

Bei der technischen Herstellung von Biodiesel stellen unter anderem die Konzentration des Katalysators, die Temperatur, die Verweilzeit, die Feuchtigkeit, die Gegenwart von freien Fettsäuren und der Alkoholüberschuss wichtige Prozessparameter für die Optimierung der Ausbeute dar (B. Freedman, E.H. Pryde, T.L. Mounts, Variables affecting the yield of fatty esters from transesterified vegetable oils, J. Am. Oil Chem. Soc., 61, 1638, 1984).

Die eingesetzten Triglyceride enthalten etwa 10 Gew.-% Glycerin, das als verunreinigte Fraktion nach der Umesterung bevorzugt durch eine Phasentrennung oder Extraktion von der Biodiesel-Fraktion isoliert wird. Beide Fraktionen werden dann in mehrstufigen Aufarbeitungsschritten von Wasser, Säuren, Katalysatoren, Alkohol, Salzen und Nebenprodukten weitestgehend befreit.

Acrolein ist ein wichtiges Zwischenprodukt und somit von großer wirtschaftlicher Bedeutung für die Darstellung von Acrylsäure, D,L-Methionin und dem Methionin-Hydroxy-Analogen (MHA,=2-Hydroxy-4-methylthiobuttersäure). Methionin ist eine essentielle Aminosäure, die u.a. als Ergänzung in Futtermitteln eingesetzt wird. Nutritivitätsverbessernde Futtermittelzusatzstoffe sind heute ein unverzichtbarer Bestandteil der Tierernährung. Sie dienen der besseren Verwertung des Nahrungsangebotes, stimulieren das Wachstum und fördern die Eiweißbildung. Einer der wichtigsten dieser Zusatzstoffe ist die essentielle Aminosäure Methionin, die vor allem in der Geflügelaufzucht als Futtermitteladditiv eine herausragende Stellung einnimmt. Auf diesem Gebiet haben, aber auch sogenannte Methionin-Ersatzstoffe wie das Methionin-Hydroxy-Analog nicht unerhebliche Bedeutung, da sie ähnliche wachstumsstimulierende Eigenschaften aufweisen wie die dafür bekannte Aminosäure.

Nach dem Stand der Technik erfolgt die Synthese von Acrolein durch heterogen katalysierte selektive Oxidation von Propen an Mischoxidkatalysatoren. EP 417723 beschreibt die Synthese an komplexen Multimetallmischoxidkatalysatoren bei Temperaturen von 300 bis 380 °C und Drücken von 1,4 bis 2,2 bar. In Ullmann's Encyclopedia of Industrial Chemistry, 6. Auflage, 1999 ist das gesamte Verfahren inklusive Aufarbeitung, bei der mehrere Nebenprodukte abgetrennt werden, beschrieben. Nachdem das Eduktgemisch aus Propen, Luft und Wasser zumindest teilweise am Katalysator umgesetzt worden ist, erfolgt zunächst das Quenchen zur Abtrennung hochsiedender Nebenprodukte wie Polymere, Acrylsäure und Essigsäure. Im anschließenden Absorber wird Acrolein ausgewaschen. Nach der Desorption zur Rückgewinnung des Absorptionsmittels wird das erhaltene Rohacrolein mehrstufig destillativ gereinigt.

Wissenschaftliche Untersuchungen zur Synthese des Acroleins aus isoliertem Glycerin sind bekannt. Es ist z.B. auch bekannt, dass sich Glycerin in Gegenwart sauerer Stoffe zu verschiedenen Produkten dehydratisieren lässt.

Gemäß Organic Synthesis I, 15-18 (1964) wird durch Behandeln eines Gemisches aus pulverförmigem Kaliumhydrogensulfat, Kaliumsulfat und Glycerin bei 190 bis 200 °C Acrolein in einer Ausbeute zwischen 33 und 48 % gewonnen. Aufgrund der niedrigen Ausbeuten und der hohen Salzfrachten eignet sich dieses Verfahren jedoch nicht für den technischen Maßstab.

Im Rahmen der Untersuchungen von Modellsubstanzen von Biomasse-Pyrolyseölen wurde auch die katalytische Behandlung von Glycerin an H-ZSM5-Zeolithen bei 350 bis 500 °C untersucht - siehe Dao, Le H. et al. ACS Symp. Ser.: 376 (Pyrolysis Oils Biomass) 328-341 (1988). Kohlenwasserstoffe werden nur in geringen Ausbeuten gebildet, aber es wird auf die Bildung von Acrolein hingewiesen.

WO 2006/092272 offenbart ein Verfahren zur Herstellung von Acrylsäure bzw. von Acrylsäure-Polymeren durch Dehydratisierung von Glycerin zu einem Acrolein aufweisenden Dehydratisierungsprodukt, Gasphasenoxidation und anschließende Isolierung von Acrylsäure sowie nachfolgende Polymerisation. Ein Verfahren zur gezielten Acroleinherstellung wird jedoch nicht beschrieben.

In der DE 42 38 493 ist die sauer katalysierte Umsetzung von Glycerin zu Acrolein in der Gas- und in der Flüssigphase beschrieben. Die DE 42 38 492 betrifft ferner die Synthese von 1,2- und 1,3-Propandiol durch Dehydratisierung von Glycerin mit hohen Ausbeuten. Das dabei eingesetzte Glycerin liegt meist rein oder in Form einer wässrigen Lösung vor.

WO 2005/021697A beschreibt ein Verfahren zur Herstellung von Fettsäure-alkylestern und Glycerin durch Umsetzung eines Triglycerids, in Gegenwart eines geeigneten Katalysator mit z.B. Methanol zum entsprechenden Methylester und Glycerin. Die Aufarbeitung des Reaktionsgemisches durch destillative Verfahrensschritte und Phasentrennung ermöglicht schließlich die Isolierung des Fettsäuremethylesters und des Glycerins.

Das in der Biodiesel-Produktion nach der Phasentrennung anfallende Glycerin ist jedoch in der Regel von geringem Wert, da stark verunreinigt, z.B. durch überschüssiges Methanol, Katalysator und Seifen.

Bisher wurden die oben beschriebenen Schritte noch niemals kombiniert, .um Triglyceride direkt zur simultanen Herstellung von Fettsäurealkylestern insbesondere von Biodiesel und Acrolein einzusetzen.

Einerseits entstehen durch die separate Herstellung von Biodiesel aus Triglyceriden und Acrolein aus Propen oder Glycerin ein vergleichsweise hoher apparativer Aufwand und damit entsprechende Investitionskosten, da Synergien bei der gemeinsamen Herstellung im Verbund nicht genutzt werden. Weiterhin entstehen ein entsprechender logistischer Aufwand und damit entsprechend hohe variable Kosten wie beispielsweise Transport- und Energiekosten, um Glycerin zu befördern.

Andererseits ist der Nachteil der bisherigen klassischen Acroleinproduktion durch selektive Oxidation aus Propen insbesondere im aufwändigen Verfahren zu sehen, bei dem Propen in der Gasphase hergestellt und in der mehrstufigen Aufarbeitung isoliert werden muss sowie darin, dass Propen ein vergleichsweise teurer Ausgangsstoff ist, dessen Kosten zudem aktuell überproportional steigen.

Es war daher Aufgabe dieser Erfindung, ein Verfahren zur gleichzeitigen Herstellung von Acrolein und Fettsäurealkylestern, insbesondere von Biodiesel, aus Triglyceriden bereitzustellen und so die Nachteile des Standes der Technik zu vermeiden.

Insbesondere war es die Aufgabe, ein Verfahren aufzufinden, bei dem die primäre Umesterung von Triglyceriden auf einfache und effiziente Weise mit preiswerten und wohlfeilen Katalysatoren gelingt sowie das primär erhaltene Glycerin in effizienter Weise vom Fettsäurealkylester abgetrennt werden kann. Das abgetrennte Glycerin sollte zudem möglichst direkt und auf wiederum einfache und effiziente Weise katalytisch zu Acrolein dehydratisiert werden können und damit die Nachteile des Standes der Technik vermieden werden.

Eine weitere Aufgabe in diesem Zusammenhang war es, möglichst effiziente, gut handhabbare Katalysatoren aufzufinden, welche möglichst sowohl für die primäre Umesterung (Biodiesel- bzw. Glycerin-Bildung) als auch für die sekundär ablaufende Dehydratisierung des Glycerins zum Acrolein geeignet sind.

Eine zusätzlich Aufgabe richtet sich darauf, ein Verfahren bereitzustellen, bei dem eine wässrige Acroleinlösung erhalten wird, die unmittelbar ihrer Verwendung, insbesondere als Ausgangsstoff zur Herstellung von Methioninverbindungen oder von Acrylsäurederivaten zugeführt werden kann.

### Beschreibung der Erfindung

Gelöst werden diese sowie weitere nicht explizit genannten Aufgaben, die jedoch aus den hierin diskutierten Zusammenhängen ohne weiteres ableitbar oder erschließbar sind durch ein Verfahren gemäß Anspruch 1. Zweckmäßige Ausformungen und Abwandlungen des erfindungsgemäßen Verfahrens werden in den auf Anspruch 1 direkt oder indirekt rückbezogenen Unteransprüchen unter Schutz gestellt.

Dadurch, dass man ein Verfahren zur Herstellung von Fettsäurealkylestern der allgemeinen Formel I: 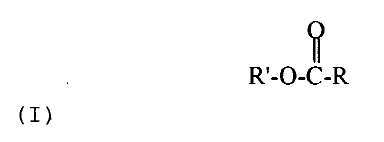 und Acrolein durch Umsetzung von Triglyceriden der allgemeinen Formel II: 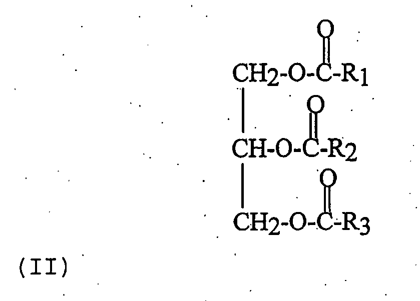 anwendet, das dadurch gekennzeichnet ist, dass
a) die Triglyceride mittels Alkoholen R'-OH in Gegenwart eines geeigneten Katalysators zum Fettsäürealkylester und Glycerin umgesetzt werden und
b) das entstandene Glycerin katalytisch zu Acrolein dehydratisiert wird,
wobei R für R₁, R₂ und R₃ steht und R₁, R₂ und R₃ allesamt gleich oder teilweise gleich oder allesamt verschieden sind und jeweils einen geradkettigen oder verzweigten und ggf. ein- oder mehrfach ungesättigten C₁₀-C₃₀-Alkyl-Rest, bevorzugt C₁₂-C₂₀-Alkyl-Rest bedeuten und R' einen C₁-C₁₀-Alkyl-Rest, bevorzugt C₁-C₆-Alkyl-Rest, einen C₃-C₆-Cycloalkyl-Rest, bevorzugt C₆-Cycloalkyl-Rest bedeutet, und wobei die Reaktionen a) und b) gleichzeitig in einem Schritt durchgeführt werden gelingt es, die oben näher genannten Nachteile des Standes der Technik vermeiden.

Besonders bevorzugt werden Triglyceride im Reaktionsschritt. a) eingesetzt, bei denen R₁, R₂ und R₃ jeweils ein C₁₂-C₁₈-Alkyl-Rest ist.

Vorzugsweise eingesetzt werden darüber hinaus im Reaktionsschritt a) Alkohole R'-OH bei denen R'= Methyl oder Ethyl ist. Ganz besonders bevorzugt wird Methanol eingesetzt, da die so entstehenden Fettsäüremethylester am häufigsten als Biodiesel verwendet werden.

Die simultane Herstellung beider Zielprodukte kann durch verschiedene Varianten des erfindungsgemäßen Verfahrens erreicht werden. Dabei können zwei Hauptvarianten unterschieden werden.

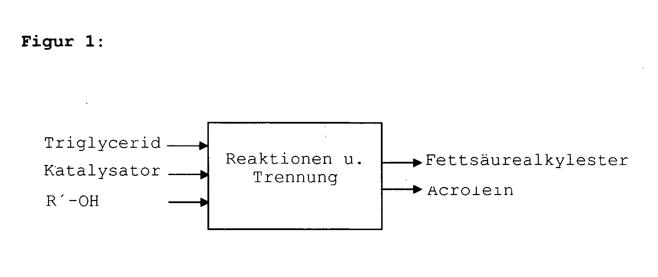

Die erste Variante umfasst ein Verfahren bei denen die Reaktionen a) und b) gleichzeitig in einem Schritt, also einstufig durchgeführt werden und ist in Figur 1 dargestellt.

Durch Verwendung eines geeigneten Katalysators oder eines Gemisches aus Katalysatoren laufen beide Reaktionen parallel ab. Dabei wird zum Beispiel ein Triglycerid-Katalysator-Gemisch, gegebenenfalls in Gegenwart eines Lösungsmittels in der Flüssigphase an einem sauren Katalysator mit dem Alkohol R' OH umgesetzt.

Für die Reaktionen a) und b) können ein homogener oder heterogener saurer Katalysator und/oder Salze von Mineralsäuren oder ggf. ein geeigneter Biokatalysator verwendet werden.

Bevorzugt sind dabei Verfahren unter Verwendung eines homogenen sauren Katalysators, insbesondere eines homogenen Katalysators mit einem pKs-Wert < 7.

Als Katalysatoren geeignet sind beispielsweise starke Brönstedt-Säuren wie Schwefelsäure, Phosphorsäure, Toluolsulfonsäure oder Methansulfonsäure.

Als salzartige Katalysatoren geeignet sind beispielsweise Kalium-, Natrium- oder Cäsiumsulfat, Kalium-, Natrium- oder Cäsiumhydrogensulfat oder Mischungen der genannten Hydrogensulfate oder Sulfate, Lithiumphosphat, Eisenphosphat, Zinksulfat ggf. in homogener Phase und ggf.in Anwesenheit eines Lösungsmittels.

Bei Verwendung eines heterogenen sauren Katalysators sind solche bevorzugt mit einem H₀-Wert von < +2, vorzugsweise < -3.

Der H₀-Wert entspricht der Säurefunktion nach Hammet und lässt sich, durch die sogenannte Amintitration unter Verwendung von Indikatoren oder durch Adsorption einer gasförmigen Base ermitteln (siehe Studies in surface science and catalysis, Vol. 51, 1989: "New solid acids and bases, their catalytic properties", K. Tanabe et al., Kapitel 2, insbesondere Seiten 5-9).

Kapitel 1, Seiten 1-3 des vorgenannten Dokuments nennt zahlreiche feste Säuren, aus welchen der Fachmann, gegebenenfalls nach Bestimmung des H₀-Werts, den geeigneten Katalysator auswählen kann.

Es eignen sich als heterogener Katalysator vorzugsweise Zeolithe, Festkörpersäuren, unedle Mischoxidkatalysatoren oder saure Ionenaustauscherharze.

Als heterogener Katalysator bevorzugt werden dabei:
(i) natürliche oder synthetische silikatische Stoffe wie insbesondere Mordenit, Montmorillonit und saure Zeolithe, wie beispielsweise HZSM-5, MCM-22, Zeolith Beta;
(ii) mit ein-, zwei- oder mehrbasigen anorganischen Säuren insbesondere Phosphorsäure, Schwefelsäure oder sauren Salzen anorganischer Säuren belegte Trägermaterialien, wie oxidische oder silikalitische Stoffe, vorzugsweise Aluminiumoxid, Titanoxid, Siliziumdioxid, Zirkondioxid oder deren Mischungen;
(iii) Oxide und Mischoxide, wie beispielsweise Aluminiumoxide, Zinkoxid-Aluminiumoxid-Mischungen oder Heteropolysäuren, oder
(iv) Polystyrolsulfonsäureharze, insbesondere Lewatit- oder Amberlite-Harze oder perfluorierte Polymersulfonsäureharze, insbesondere Nafion.

Als Biokatalysatoren eignen sich Lipasen, Esterasen, Hydratasen und/oder Hydrolasen.

Die Verwendung eines Lösungsmittels ist bevorzugt, denn dadurch werden die Konzentrationen an reaktiven Zwischenverbindungen gesenkt und Nebenreaktionen zu Oligomeren, Polymeren und anderen Hochsiedern minimiert. Weiterhin ist für die Esterbildung der Zusatz eines Alkohols erforderlich. Eingesetzt werden dem Fachmann bekannte Lösungs- und Verdünnungsmittel wie beispielsweise Methyl-tert.-butylether, Ethyl-tert.-butylether, Tetrahydrofuran, 1,4-Dioxan, Diglyme, Toluol, Methylisobutylketon und/oder der zur Umesterung benutzte Alkohol R'-OH. Auch die eingesetzten Triglyceride können als Lösungsmittel wirken, ebenso das intermediär entstehende Glycerin sowie die entstehenden Fettsäurealkylester und bei der Dehydratisierung entstehendes Wasser.

Das Lösungsmittel wird vorzugsweise im Überschuss zugegeben und ist bevorzugt mit dem für die Umesterung eingesetzten Alkohol R'-OH identisch. Methanol und/oder Ethanol werden dabei ganz besonders bevorzugt verwendet.

Es wird eine Reaktionstemperatur zwischen 30 und 500 °C, bevorzugt zwischen 50 und 350 °C, besonders bevorzugt zwischen 70 und 300 °C gearbeitet. Der Druck wird dabei derart eingestellt, dass der flüssige Zustand des Reaktionsgemisches erhalten bleibt. Üblicherweise beträgt der Druck zwischen 1 und 300 bar, bevorzugt zwischen 1 und 150 bar, besonders bevorzugt zwischen 1 und 50 bar.

Die Durchführung kann in dem vom Fachmann bekannten Reaktionsgefäßen wie beispielsweise Festbettreaktoren, Rührkesseln, Strömungsrohren oder Kombinationen durchgeführt werden.

Gebildetes Acrolein kann in an sich bekannter Weise allein oder zusammen mit einem Teil des Lösungs- oder Verdünnungsmediums und/oder Wasser bevorzugt durch Strippen oder Destillation aus dem erhaltenen Reaktionsgemisch abgetrennt werden. Nicht umgesetzte Intermediate oder Glycerin verbleiben in der Reaktionsstufe und werden fortlaufend weiterumgesetzt. Durch Aufangen des abgetrennten Acroleins mit Wasser (Quenchen) wird eine wässrige Acroleinlösung erhalten, die unmittelbar der Verwendung oder einer weiteren Aufreinigung zugeführt werden kann.

Je nach Verwendungszweck können dementsprechend Verunreinigungen oder Nebenprodukte im Acrolein toleriert werden oder sind durch Aufarbeitungsschritte zu entfernen. Bevorzugt werden Wasser, Acetaldehyd, Formaldehyd oder Essigsäure sowie weitere unreaktive Nebenprodukte nicht vom Acrolein isoliert.

Die vorliegende Verfahrensvariante ist folglich dadurch gekennzeichnet, dass das in der Reaktion b) gebildete Acrolein aus dem Reaktionsgemisch allein oder ggf. zusammen mit wenigstens einem Teil des Lösungsmittels und/oder des Wassers abgetrennt wird.

Dabei kann das Acrolein durch Strippen, Destillation, Extraktion, Phasentrennung oder Einsatz von Membranen aus dem Reaktionsgemisch abgetrennt werden.

Ganz besonders bevorzugt wird dabei das Acrolein während der Reaktion durch Destillation aus dem Reaktionsgemisch abgetrennt.

Für die simultane Synthese von Acrolein und Biodiesel aus Triglyceriden ist neben der Beteiligung von Dehydratisierungsschritten die selektive Umesterung erforderlich. Da bei der Dehydratisierung zu Acrolein Wasser gebildet wird, das bei der Fettsäurealkylester-Synthese ebenfalls zur unerwünschten Entstehung der freien Fettsäure führen kann, sollte neben Glycerin, das im wesentlichen durch die Folgereaktion zum Acrolein verbraucht wird, auch bevorzugt Wasser aus dem Reaktionsgemisch bzw. Gleichgewicht entfernt werden. Dies kann direkt im ersten Reaktionsschritt oder in einer nachgeschalteten Stufe erfolgen.

Die gleichzeitige Entfernung des Acroleins während der chemischen Umsetzungen aus dem Reaktionsgemisch ist mittels Destillation relativ einfach möglich und daher bevorzugt. Dies ist durch die großen Unterschiede der Siedepunkte von Acrolein einerseits und Triglyceriden, Fettsäurealkylestern und Glycerin andererseits bedingt. Durch die sofortige Abtrennung des entstandenen Acroleins aus dem Reaktionsgemisch kann zudem die Ausbeute erhöht werden, da Acrolein im Reaktionsgemisch eine sehr reaktive Verbindung darstellt, die zu unerwünschten Nebenreaktionen führen kann. Die gute destillative Abtrennbarkeit des Acroleins von der Fettsäurealkylester enthaltenden Reaktionsmischung wird am Beispiel 2 deutlich, bei dem Acrolein quantitativ durch Vakuumdestillation vom Biodiesel abgetrennt wurde.

In einer weiteren Ausführungsform der Erfindung wird Acrolein nicht sofort zu Reaktionsbeginn der Umesterung gebildet, sondern erst gebildetes Glycerin abgetrennt, nachdem der Großteil der Triglyceride zu Fettsäurealkylestern umgesetzt wurde. Das nach der Umesterung entstandene Glycerin wird dann mit Hilfe eines Dehydratisierungskatalysators zu Acrolein umgesetzt. Die Katalysatoren können aus den beschriebenen sauren oder basischen Komponenten oder aus dem Fachmann bekannten Komponenten von Dehydratisierungskatalysatoren bestehen.

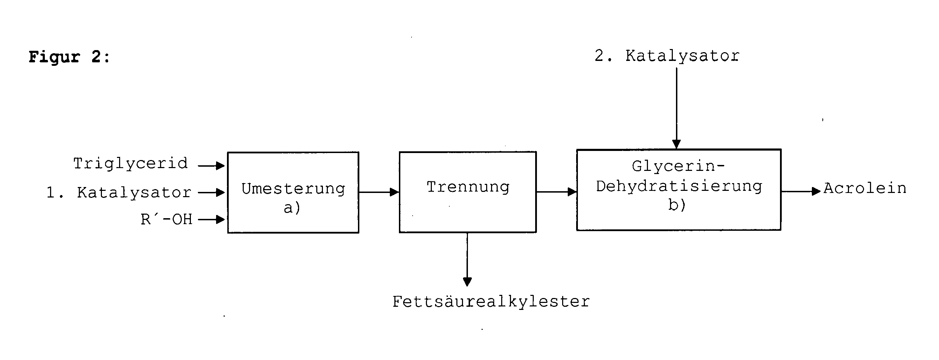

Diese weitere Verfahrensvariante umfasst ein Verfahren, bei dem die Reaktionen a) und b) in getrennten Schritten durchgeführt werden und ist in Figur 2 dargestellt. Dabei wird zunächst die Reaktion a) (Umesterung) von Triglycerid mit Alkohol R'OH zu Glycerin und Fettsäurealkylester (Biodiesel) in Gegenwart eines geeigneten ersten Katalysators durchgeführt. Anschließend erfolgt eine Trennung in eine Glycerin-haltige und eine Fettsäurealkylester-haltige Phase. Die Reaktionen a) und b) werden bei dieser Verfahrensvariante gleichzeitig in räumlich getrennten Schritten durchgeführt und das primär gebildete Glycerin aus dem Reaktionsgemisch der Reaktion a) ganz oder teilweise abgetrennt durch Membrantrennung, durch Phasentrennung nachdem sich eine Fettsäurealkylester enthaltende lipophile Phase A und eine Glycerin-haltige hydrophile Phase B gebildet haben oder durch Reaktivdestillation und in Reaktion b) katalytisch zu Acrolein dehydratisiert. Das Glycerin in der abgetrennten Glycerin-haltigen Phase wird in Gegenwart eines geeigneten zweiten Katalysators zu Acrolein dehydratisiert (Reaktion b). Der Fettsäurealkylester kann direkt oder nach geeigneter Nachbehandlung wie z.B. einer weitgehenden Entwässerung als Brennstoff bzw. Biodiesel verwendet werden.

Durch die Verwendung des Dehydratisierungskatalysators in einem getrennten Reaktionsschritt wird die Bildung reaktiver Intermediate vermieden, die sich bei den oben angegebenen Bedingungen zumindest aus Teilmengen der Triglyceride oder des Glycerins bilden können. Die reaktiven Intermediate bilden sonst verstärkt unerwünschte Nebenprodukte, die zu einer Desaktivierung des Katalysators oder zur Verminderung der Ausbeute führen. Werden diese Intermediate minimiert, kann die Ausbeute zu Acrolein und Fettsäurealkylestern verbessert werden.

Dies kann einerseits in einer diskontinuierlichen Fahrweise erfolgen, wobei der Dehydratisierungskatalysator nach einer gewissen Reaktionszeit dem dann zweiphasigen Reaktionsgemisch zugesetzt wird. Geeignet ist dafür beispielsweise die Durchführung im Rührkessel.

Andererseits kann der Dehydratisierungskatalysator erst an einem vom Reaktoreingang in Strömungsrichtung entfernten Ort oder in einem weiteren Reaktorabschnitt eingespeist werden, nachdem eine Phasentrennung erfolgt ist. Bis die Glycerinlösung und der Katalysator diesen Ort erreichen, kann bereits ein Teil des Glycerins zu reaktiven Intermediaten umgesetzt sein. Diese Fahrweise kann technisch beispielsweise in einer Rührkesselkaskade oder einem Strömungsrohr realisiert werden. Die Phasentrennung kann bei dieser Ausführungsform der Erfindung durch nachträgliche Zugabe von Wasser oder durch destillative Entfernung von überschüssigem Alkohol beschleunigt werden, was die anschließende Dehydratisierung in der Glycerinphase erleichtert.

Ein weiterer Vorteil der Dehydratisierung in einem zweiten Schritt besteht darin, dass die Ausbeuten an Acrolein und Fettsäurealkylestern gesteigert werden können, weil ein anhand des Reaktionsverhaltens der Reaktanden optimiertes Temperaturprofil bzw. Temperaturprogramm für jeden Schritt separat eingestellt werden kann. So erfordert die Umesterung der Triglyceride eine deutlich niedrigere Aktivierungsenergie oder Reaktionstemperatur als die Aktivierung von Glycerin und dessen erster Dehydratisierungsschritt zur schnellen Herstellung von Zwischenprodukten wie z.B. Hydroxypropionaldehyd. Die entstandenen Fettsäurealkylester tendieren hingegen bei höheren Temperaturen von ca. 150 bis 250 °C zu unselektiven Weiterreaktionen. Somit stellt die Durchführung der gemeinsamen Herstellung von Fettsäurealkylestern und Acrolein mittels Durchlaufen von zwei Reaktionsstufen bei unterschiedlichen Reaktionsbedingungen eine bevorzugte Ausführungsform dar.

Eine bevorzugte Ausführungsform des Verfahrens ist dadurch gekennzeichnet, dass die Umesterungsreaktion a) mit Hilfe eines alkalischen Katalysators durchgeführt wird, vorzugsweise mit einem alkalischen Katalysator, bei dem der pKs-Wert der korrespondierenden Säure > 13 ist.

Hier sind insbesondere Alkalialkoholate des für die Umesterung eingesetzten Alkohols R'OH zu nennen. Bevorzugt werden dabei die Alkalialkoholate der C₁-C₄-Alkohole, insbesondere Alkalimethylate oder -ethylate und ganz besonders bevorzugt Natriummethylat.

Die Möglichkeit, alkalische Katalysatoren für die Umesterung in Reaktion a) einzusetzen, stellt bei der zweiten Verfahrensvariante einen zusätzlichen Vorteil dar, weil hier durch die nachfolgende Abtrennung der GlycerinPhase praktische keine Reaktion mit den in der Dehydratisierung zu verwendenden sauren Katalysatoren stattfinden kann.

Ebenso wie bei der Reaktionsführung in einem Schritt ist es aber auch hier möglich, dass Reaktion a) mit Hilfe eines homogenen oder heterogenen sauren Katalysators durchgeführt wird. Dieser Katalysator kann zudem verschieden sein von dem in der Dehydratisierung zu verwendenden sauren Katalysator, was die Variationsbreite und Flexibilität des Verfahrens weiter erhöht.

Bei Verwendung eines homogenen sauren Katalysators in Reaktion a), werden bevorzugt Katalysatoren mit einem pKs-Wert < 7 verwendet.

Bei Verwendung eines heterogenen sauren Katalysators in Reaktion a), werden bevorzugt Katalysatoren mit einem H₀-Wert < +2, vorzugsweise < -3 verwendet.

Sowohl die alkalisch als auch die sauer katalysierte Umesterung a) wird bei Temperaturen von 20 bis 150°, vorzugsweise 25 bis 100°C, bevorzugt von 25 bis 80°C durchgeführt.

Auf diese Weise ist es möglich durch Wahl von optimalen Reaktionsbedingungen, das Glycerin neben Biodiesel in einer ersten Reaktionsstufe zu bilden und anschließend in einer zweiten Stufe selektiv zu Acrolein umzusetzen.

Erfindungsgemäß ist auch möglich, dass Reaktion a) mit Hilfe eines Biokatalysators durchgeführt wird.

Als Biokatalysatoren werden dabei vorzugsweise Lipasen, Esterasen, Hydratasen oder Hydrolasen verwendet.

Bei der Verfahrensvariante mit getrennten Reaktionsschritten a) und b) wird das primär gebildete Glycerin aus dem Reaktionsgemisch der Reaktion a) ganz oder teilweise abgetrennt und bei der getrennt ablaufenden Reaktion b) katalytisch zu Acrolein dehydratisiert.

Für die Dehydratisierungsreaktion b) kann erfindungsgemäß ein homogener oder heterogener saurer Katalysator verwendet werden.

Im Falle von homogenen Katalysatoren sind solche mit einem pKs-Wert < 7 bevorzugt.

Insbesondere können hier Schwefelsäure, Phosphorsäure, Toluolsulfonsäure oder Methansulfonsäure als Katalysator verwendet werden.

Desweiteren werden bevorzugt als homogene Katalysatoren für die Dehydratisierungsreaktion b) Salze von Mineralsäuren ggf. in Anwesenheit eines Lösungsmittels verwendet.

Ganz besonders bevorzugt werden dabei Kalium-, Natrium- oder Cäsiumsulfat, Kalium-, Natrium- oder Cäsiumhydrogensulfat oder Mischungen der vorstehend genannten Hydrogensulfate und Sulfate, Lithiumphosphat, Eisenphosphat, Zinksulfat, ggf. in Anwesenheit eines Lösungsmittels, als Katalysator verwendet.

Im Falle von heterogenen Katalysatoren sind solche mit einem H₀-Wert < +2 , vorzugsweise < -3 bevorzugt.

Bevorzugt werden Zeolithe, Festkörpersäuren, unedle Mischoxidkatalysatoren oder saure Ionenaustauscherharze als heterogener Katalysator verwendet.

Als heterogener Katalysator eignen sich insbesondere:
(i) natürliche oder synthetische silikatische Stoffe, wie insbesondere Mordenit, Montmorillonit und saure Zeolithe, insbesondere HZSM-5, MCM-22, Zeolith Beta;
(ii) mit ein-, zwei- oder mehrbasigen anorganischen Säuren insbesondere Phosphorsäure, Schwefelsäure oder sauren Salzen der anorganischen Säuren, belegte Trägermaterialien, wie beispielsweise oxidische oder silikatische Stoffe, insbesondere Aluminiumoxid, Titandioxid, Siliziumdioxid, Zirkondioxid oder deren Mischungen;
(iii) Oxide und Mischoxide, insbesondere Aluminiumoxide, Zinkoxid-Aluminiumoxid-Mischungen oder Heteropolysäuren oder
(iv) Polystyrolsulfonsäureharze, insbesondere Lewatit- oder Amberlite-Harze, oder perfluorierte Polymersulfonsäureharze, insbesondere Nafion.

Die vorzugsweise einzustellenden Reaktionsbedingungen entsprechen denen, die bei der oben beschriebenen ersten einstufigen Verfahrensvariante angewendet werden. Lediglich die Reaktionstemperatur der zweiten Reaktion, der Dehydratisierung b), liegt im Vergleich zur ersten Reaktion, der Umesterung a), bei höheren Temperaturen von 50 bis 400 °C, bevorzugt 100 bis 350 °C.

Ebenso bevorzugt wird die Dehydratisierungsreaktion b) mit Hilfe eines Biokatalysators durchgeführt.

Als Biokatalysator verwendet werden dabei beispielsweise Dehydratasen, Hydratasen oder Hydrolasen.

Der Reaktionsschritt b) wird dabei vorzugsweise in Gegenwart eines Lösungsmittels durchgeführt.

Als Lösungsmittel bevorzugt werden Methyl-tert.-butylether, Ethyl-tert.-butylether, Tetrahydrofuran, 1,4-Dioxan, Diglyme, Toluol, Methylisobutylketon und/oder der zur Umesterung in Reaktionsschritt a) bereits benutzte Alkohol R'-OH eingesetzt.

Eine Möglichkeit dabei ist, dass das Glycerin aus dem Reaktionsgemisch a) durch Destillation abgetrennt wird.

Durch eine Vakuumdestillation des Reaktionsgemisches a) kann das Glycerin in einer dem ersten Reaktionsapparat aufgesetzten Destillationskolonne abgetrennt werden.

Eine besonders vorteilhafte Ausführungsform des Verfahrens besteht darin, dass Glycerin aus dem Reaktionsgemisch a) durch Reaktivdestillation abgetrennt wird und dabei am gleichzeitig vorhandenen sauren Katalysator zu Acrolein dehydratisiert wird (Reaktionsschritt b).

In diesem Fall befindet sich in der Destillationskolonne ein heterogener Dehydratisierungskatalysator vorzugsweise der o.g. näher bezeichneten Art (i) bis (iv), der gleichzeitig die Umsetzung des abzutrennenden Glycerins zu Acrolein beschleunigt. Die Temperatur in der nachgeschalteten Kolonne beträgt zwischen 50 und 300 °C, bevorzugt zwischen 80 und 250 °C, besonders bevorzugt zwischen 100 und 200 °C. Der Druck beträgt dort zwischen 0,1 und 1500 mbar, bevorzugt zwischen 0,5 und 1000 mbar, besonders bevorzugt zwischen 2 und 100 mbar.

Der feste, in der Regel unlösliche saure Dehydratisierungskatalysator ist innerhalb der Destillationskolonne als Schüttung oder als Beschichtung eingebracht.

Der Vorteil der Durchführung der Reaktionen a) und b) in zwei getrennten Schritten besteht auch darin, dass die Stabilität des Prozesses aufgrund abgegrenzter Verfahrensschritte bei kontinuierlicher Betriebsweise verbessert ist.

Neben der Destillation bzw. Reaktivdestillation können noch weitere Möglichkeiten zur Abtrennung des Glycerins verwendet werden.

Eine weitere bevorzugte Ausführungsform des erfindungsgemäßen Verfahrens ist dadurch gekennzeichnet, dass Glycerin aus der Reaktionslösung durch einfache Phasentrennung abgetrennt wird, nachdem sich eine Fettsäurealkylester enthaltende lipophile Phase A und eine Glycerin-haltige hydrophile Phase B gebildet haben.

Diese Phasentrennung kann darüberhinaus durch Zugabe eines Phasentrennmittels ermöglicht oder gefördert werden.

Bevorzugte Phasentrennmittel sind dabei Wasser und/oder ein organisches Lösungsmittel und/oder Fettsäurealkylester.

Die gemeinsame Herstellung von Acrolein und Fettsäurealkylestern kann erfindungsgemäß auch dadurch erreicht werden, dass die Phasentrennung in eine Triglycerid und Fettsäurealkylester enthaltende lipophile Phase A und in eine Wasser- und Glycerin-haltige hydrophile Phase B bereits zu Beginn der Umesterung bewusst gefördert wird und die Reaktionen a) und b) parallel in den beiden verschiedenen Flüssigphasen ablaufen (Zweiphasen-Katalyse). Dies kann einerseits durch die Zugabe von Wasser oder anderen Lösungsmitteln erfolgen.

Andererseits kann der für die Umesterung eingesetzte überschüssige Alkohol destillativ entfernt werden, um die Phasentrennung zu beschleunigen. Dies hat den Vorteil, dass für die Umesterungsreaktion a) und für die Dehydratisierungsreaktion b) jeweils optimierte Katalysatoren eingesetzt werden können. Beide Reaktionen laufen zudem getrennt in den jeweiligen Phasen ab, ohne sich gegenseitig zu beeinflussen.

Bevorzugt ist daher ein Verfahren, bei dem die Reaktion a) in der lipophilen Phase A, die Triglycerid und Fettsäurealkylester enthält, durchgeführt wird und die Reaktion b) in der hydrophilen Phase B, die Glycerin, Wasser und Acrolein enthält.

Der leichte Übergang des Acroleins aus der Fettsäurealkylester enthaltenden lipophilen Phase A in die wässrige hydrophile Phase B wird am Beispiel 1 deutlich, bei dem Acrolein überwiegend in der wässrigen Phase wiedergefunden wurde.

Somit kann die Ausbeute beider Zielprodukte insgesamt gesteigert werden, indem weniger unselektive Nebenreaktionen ablaufen. Es wird auch angestrebt Restfeuchte oder zugegebenes Wasser ausschließlich in der Glycerinphase zu binden, da es sonst verstärkt zur unerwünschten Bildung von freien Fettsäuren führt.

Die Katalysatorsysteme für die Umesterung und für die Dehydratisierung sind im Fall der Zweiphasen-Katalyse so abgestimmt, dass sich bei Verwendung von homogenen Katalysatoren der Umesterungskatalysator in der Triglyceridphase und der Dehydratisierungskatalysator in der Glycerin- haltigen Phase lösen und nicht umgekehrt, so dass ein in der lipophilen Phase A bevorzugt löslicher Katalysator dort die Reaktion a) beschleunigt und ein in der hydrophilen Phase bevorzugt löslicher Katalysator dort die Reaktion b).

Im allgemeinen ist eine homogene Lösung für einen guten Stoffaustausch vorteilhaft, aber hier nicht zwingend erforderlich. Dies wird hier sogar durch das Prinzip der Zweiphasen-Katalyse gezielt ausgenutzt, wobei das Produkt Acrolein in der wäßrigen Glycerinphase gut löslich ist. Somit kann Acrolein ohne aufwendige Aufarbeitung einfach durch Phasentrennung oder zusätzliche Extraktion vom Fettsäurealkylester separiert und somit das Gesamtverfahren vereinfacht werden. Dies wird auch am Beispiel 1 deutlich.

Bei der Esterspaltung handelt es sich um eine Gleichgewichtsreaktion, die je nach Reaktionsbedingungen in die eine oder andere Richtung gelenkt werden kann. Dies wird bei der beschriebenen Ausführungsform der Zweiphasen-Katalyse gezielt ausgenutzt, indem Glycerin als Reaktionsprodukt aus.der Triglycerid/und Fettsäurealkylester enthaltenden Phase und somit aus dem Gleichgewicht entfernt wird.. Damit wird die Reaktion a) verstärkt in Richtung Fettsäurealkylester bzw. Biodiesel beschleunigt bzw. eine Rückreaktion zu Triglyceriden minimiert. Dadurch können Reaktionsbedingungen eingestellt werden, die sonst eigentlich nicht optimal für die Herstellung von Biodiesel wären bzw. kann der Bereich der geeigneter Reaktionsbedingungen erweitert werden. So kann beilspielsweise der Überschuss an Alkohol gesenkt werden.

Fettsäurealkylester der Formel I werden vorzugsweise aus der lipophilen Phase A gewonnen und können direkt oder ggf. nach weiterer Aufreinigung als Brennstoff verwendet werden.

Acrolein wird aus der hydrophilen Phase B gewonnen und kann direkt oder ggf. nach weiterer Aufreinigung als Ausgangsstoff für Methionin-Produkte oder Acrylsäure verwendet werden.

Eine Ausführungsform der Erfindung ist dadurch gekennzeichnet, dass die eingesetzten Triglyceride in Gegenwart von Alkoholen R'OH katalytisch in Fettsäurealkylester und Acrolein umgewandelt werden, wobei die Umesterungsreaktion a)und die Dehydratisierungsreaktion b) unter Verwendung von Membranen gleichzeitig aber räumlich getrennt durchgeführt werden (Membrantrennung). Durch die Membran werden die Triglyceride und Fettsäurealkylester selektiv zurückgehalten (Retentat), während entstehendes Glycerin in das Permeat gelangt. Dadurch erfolgt die Trennung ohne separaten Aufarbeitungsschritt, was die Herstellkosten senkt.

So kann Glycerin aus dem Reaktionsgemisch der Reaktion a) erfindungsgemäß auch durch Membranfiltration abgetrennt werden. Als Membranmaterialien eignen sich insbesondere organische oder keramische Materialien.

Die Dehydratisierungsreaktion kann auch durch geeignete auf oder in der Membran immobilisierte Katalysatoren oder durch eine separate Katalysatorzugabe in das Permeat erfolgen. Die katalytisch aktiven Komponenten für die Dehydratisierung können auch unlösliche Bestandteil der Membran sein.

Als Membranen eignen sich beispielsweise Löslichkeitsmembranen oder Diffusionsmembranen aus der Mikro- oder Ultrafiltration, die aufgrund ihrer funktionellen Gruppen, Polarität oder Porenstruktur die Triglyceride zurückhalten, aber für Glycerin durchgängig sind. Geeignete Membranen sind insbesondere aufgebaut aus organischen Materialien wie Polyethersulfonen (PES), Celluloseestern, Polyimiden oder Polyetherimiden, Polyfettsäureamiden oder Polyacrylnitril (PAN) oder aus keramischen Materialien wie Aluminiumoxid, Titandioxid, Siliciumdioxid, Zirkoniumdioxid (Baddeleyit) sowie deren Kombinationen.

Die Vorteile der beschriebenen simultanen Herstellung von Biodiesel und Acrolein bestehen in einer erhöhten Produktivität, Wertschöpfung und Zeitersparnis. Gleichzeitig werden die Transport- und Energiekosten reduziert. Insgesamt wird die Produktion von Biodiesel und Acrolein wirtschaftlicher.

Gleichzeitig können höhersiedende Nebenprodukte, die bei der Acroleinsynthese anfallen als zusätzlicher Brennwert dem Biodiesel beigemischt oder ggf. im Biodiesel belassen werden. Dies können beispielsweise Di- oder Trimere des Acroleins oder Ether aus Glycerin sein.

Gegenstand der Erfindung ist auch eine Acrolein enthaltende Mischung, hergestellt nach dem hier dargestellten erfindungsgemäßen Verfahren, sowie eine Fettsäurealkylester der Formel I enthaltende Mischung, hergestellt nach dem hier dargestellten erfindungsgemäßen Verfahren.

Die nachfolgenden Beispiele sollen die dargestellte Erfindung näher erläutern jedoch keines falls beschränkend wirken.

### Beispiele

### Beispiel 1:

50 g Biodiesel und 50 g Acrolein wurden gemischt und bildeten ein homogenes Gemisch. Im Scheidetrichter wurde dieses homogene Gemisch mit 250 g Wasser versetzt, geschüttelt und stehen gelassen, wobei sich zwei Phasen bildeten. Nach der Ausbildung der Phasengrenze wurde die untere, wässrige Phase im Scheidetrichter abgetrennt und zur Bestimmung des Acroleingehalts mittels Gaschromatographie analysiert. Insgesamt wurden in der wässrigen Phase dadurch 33,4 g Acrolein erhalten. Bereits bei einfacher, einstufiger Extraktion mit Wasser konnte somit ein großer Teil des Acroleins vom Biodiesel abgetrennt werden.

### Beispiel 2:

Im Kolben eines Rotationsverdampfers wurde ein Gemisch aus 50 g Biodiesel und 50 g Acrolein vorgelegt und in einem Wasserbad auf 35°C beheizt. Mittels einer Wasserstrahlpumpe wurde das Gemisch langsam unter Vakuum gesetzt. In der gekühlten Destillationsvorlage wurden 50 mg Hydrochinon zur Stabilisierung des destillerten Acroleins vorgelegt. Innerhalb von einer Stunde wurde ein Destillat von 49,1 g Acrolein erhalten. Die Reinheit des Destillats wurde mittels Gaschromatographie zu mindestens 99,5 % bestätigt. Im Kolben des Rotationsverdampfers blieben 50,6 g zurück.

## Patentansprüche

1. Verfahren zur Herstellung von Fettsäurealkylestern der allgemeinen Formel I: 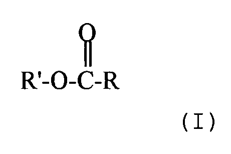 und Acrolein durch Umsetzung von Triglyceriden der allgemeinen Formel II: 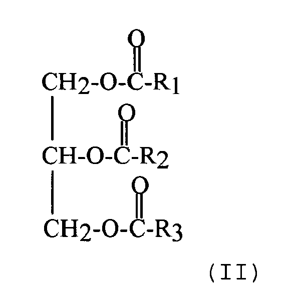 **dadurch gekennzeichnet, dass**
a) die Triglyceride mittels Alkoholen R'-OH in Gegenwart eines geeigneten Katalysators zum Fettsäurealkylester und Glycerin umgesetzt werden und
b) das entstandene Glycerin katalytisch zu Acrolein dehydratisiert wird,
wobei R für R₁, R₂ und R₃ steht und R₁, R₂ und R₃ allesamt gleich oder teilweise gleich oder
allesamt verschieden sind und jeweils einen geradkettigen oder verzweigten und ggf. ein- oder mehrfach ungesättigten C₁₀-C₃₀-Alkyl-Rest, bevorzugt C₁₂-C₂₀-Alkyl-Rest bedeuten und R' einen C₁-C₁₀-AlkylRest, bevorzugt C₁-C₆-Alkyl-Rest, einen C₃-C₆-Cycloalkyl-Rest, bevorzugt C₆-Cycloalkyl-Rest bedeutet, und wobei die Reaktionen a) und b) gleichzeitig in einem Schritt durchgeführt werden

2. Verfahren gemäß Anspruch 1, **dadurch gekennzeichnet, dass**
R₁, R₂ und R₃ = C₁₂-C₁₈-Alkyl-Rest ist, und/oder
R'= Methyl oder Ethyl ist.

3. Verfahren gemäß einem der Ansprüche 1 bis 2, **dadurch gekennzeichnet, dass** für die Reaktionen a) und b) ein homogener oder heterogener saurer Katalysator und/oder Salze von Mineralsäuren oder ein Biokatalysator verwendet wird.

4. Verfahren gemäß Anspruch 3, **dadurch gekennzeichnet, dass** ein homogener Katalysator mit einem pKs-Wert < 7 verwendet wird, vorzugsweise
Schwefelsäure, Phosphorsäure, Toluolsulfonsäure oder Methansulfonsäure.

5. Verfahren gemäß Anspruch 3, **dadurch gekennzeichnet, dass** Kalium-, Natrium- oder Cäsiumsulfat, Kalium-, Natrium- oder Cäsiumhydrogensulfat oder Mischungen der genannten Hydrogensulfate und Sulfate, Lithiumphosphat, Eisenphosphat, Zinksulfat, ggf. in Anwesenheit eines Lösungsmittels als Katalysator verwendet werden.

6. Verfahren gemäß Anspruch 3, **dadurch gekennzeichnet, dass** ein heterogener Katalysator mit einem H₀-Wert < +2 verwendet wird, vorzugsweise mit einem Hₒ-Wert < -3,
besonders bevorzugt Zeolithe, Festkörpersäuren, unedle Mischoxidkatalysatoren oder saure Ionenaustauscherharze als heterogener Katalysator.

7. Verfahren gemäß Anspruch 6, **dadurch gekennzeichnet, dass**
(i) natürliche oder synthetische silikatische Stoffe, insbesondere Mordenit, Montmorillonit und saure Zeolithe, insbesondere HZSM-5, MCM-22 und Zeolith Beta,
(ii) mit ein-, zwei- oder mehrbasigen anorganischen Säuren, insbesondere Phosphorsäure, Schwefelsäure oder sauren Salzen anorganischer Säuren belegte Trägermaterialien, wie insbesondere oxidische oder silikatische Stoffe, vorzugsweise Aluminiumoxid, Titandioxid, Siliziumdioxid, Zirkondioxid oder deren Mischungen,
(iii) Oxide und Mischoxide, insbesondere Aluminiumoxide, Zinkoxid-Aluminiumoxid-Mischungen oder Heteropolysäuren, oder
(iv) Polystyrolsulfonsäureharze, insbesondere Lewatit- oder Amberlite-Harze oder perfluorierte Polymersulfonsäureharze, insbesondere Nafion, verwendet werden.

8. Verfahren gemäß Anspruch 3, **dadurch gekennzeichnet, dass** Lipasen, Esterasen, Hydratasen und/oder Hydrolasen als Biokatalysator verwendet werden.

9. Verfahren gemäß einem der Ansprüche 1 bis 8, **dadurch gekennzeichnet, dass** die Reaktion in Gegenwart eines Lösungsmittels durchgeführt wird, vorzugsweise
Methyl-tert.-butylether, Ethyl-tert.-butylether, Tetrahydrofuran, 1,4-Dioxan, Diglyme, Toluol, Methylisobutylketon und/oder der zur Umesterung gemäß Anspruch 1 benutzte Alkohol R'-OH.

10. Verfahren gemäß einem der Ansprüche 1 bis 9, **dadurch gekennzeichnet, dass** das in Reaktion b) gebildete Acrolein aus dem Reaktionsgemisch allein oder ggf. zusammen mit wenigstens einem Teil des Lösungsmittels abgetrennt wird, bevorzugt
durch Strippen, Destillation, Extraktion, Phasentrennung oder Einsatz von Membranen, ganz besonders bevorzugt das Acrolein bereits während der Reaktion durch Destillation aus dem Reaktionsgemisch abgetrennt wird.

11. Verfahren zur Herstellung von Fettsäurealkylestern der allgemeinen Formel I: 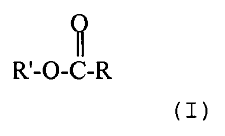 und Acrolein durch Umsetzung von Triglyceriden der allgemeinen Formel II: 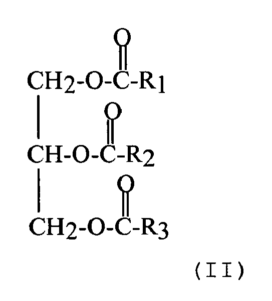 **dadurch gekennzeichnet, dass**
a) die Triglyceride mittels Alkoholen R'-OH in Gegenwart eines geeigneten Katalysators zum Fettsäurealkylester und Glycerin umgesetzt werden und
b) das entstandene Glycerin katalytisch zu Acrolein dehydratisiert wird,
wobei R für R₁, R₂ und R₃ steht und R₁, R₂ und R₃ allesamt gleich oder teilweise gleich oder
allesamt verschieden sind und jeweils einen geradkettigen oder verzweigten und ggf. ein- oder mehrfach ungesättigten C₁₀-C₃₀-Alkyl-Rest, bevorzugt C₁₂-C₂₀-Alkyl-Rest bedeuten und R' einen C₁-C₁₀-AlkylRest, bevorzugt C₁-C₆-Alkyl-Rest, einen C₃-C₆-Cycloalkyl-Rest, bevorzugt C₆-Cycloalkyl-Rest bedeutet, und wobei die Reaktionen a) und b) gleichzeitig in räumlich getrennten Schritten durchgeführt werden und das primär gebildete Glycerin aus dem Reaktionsgemisch der Reaktion a) ganz oder teilweise abgetrennt wird durch Membrantrennung, durch Phasentrennung nachdem sich eine Fettsäurealkylester enthaltende lipophile Phase A und eine Glycerin-haltige hydrophile Phase B gebildet haben oder durch Reaktivdestillation und in Reaktion b) katalytisch zu Acrolein dehydratisiert wird.

12. Verfahren gemäß Anspruch 11, **dadurch gekennzeichnet, dass** Reaktion a) mit Hilfe eines alkalischen Katalysators durchgeführt wird, bei dem der pKs-Wert der korrespondierenden Säure > 13 ist,
dass vorzugsweise als alkalischer Katalysator Alkalialkoholate des für die Umesterung eingesetzten Alkohols R'OH oder Alkalimethylate oder -ethylate, besonders bevorzugt Natriummethylat verwendet werden.

13. Verfahren gemäß Anspruch 11, **dadurch gekennzeichnet, dass** Reaktion a) mit Hilfe eines homogenen oder heterogenen sauren Katalysators durchgeführt wird,
vorzugsweise ein homogener saurer Katalysator mit einem pKs-Wert < 7 verwendet wird, oder
**dadurch gekennzeichnet, dass** ein heterogener Katalysator mit einem H₀-Wert < +2, vorzugsweise < -3 verwendet wird.

14. Verfahren gemäß Anspruch 11, **dadurch gekennzeichnet, dass** Reaktion a) mit Hilfe eines Biokatalysators durchgeführt wird, vorzugsweise
einer Lipase, Esterase, Hydratase
oder Hydrolase.

15. Verfahren gemäß Anspruch 11-14, **dadurch gekennzeichnet, dass** für die Dehydratisierungsreaktion b) ein homogener oder heterogener saurer Katalysator verwendet wird,
bevorzugt ein homogener Katalysator mit einem pKs-Wert < 7, ganz besonders bevorzugt
Schwefelsäure, Phosphorsäure, Toluolsulfonsäure oder Methansulfonsäure.

16. Verfahren gemäß Anspruch 11-14, **dadurch gekennzeichnet, dass** für die Dehydratisierungsreaktion b) Salze von Mineralsäuren als Katalysatoren verwendet werden, vorzugsweise
Kalium-, Natrium- oder Cäsiumsulfat, Kalium-, Natrium- oder Cäsiumhydrogensulfat oder Mischungen der vorstehend genannten Hydrogensulfate und Sulfate, Lithiumphosphat, Eisenphosphat, Zinksulfat, ggf. in Anwesenheit eines Lösungsmittels , oder
dass für die Dehydratisierungsreaktion b) ein heterogener Katalysator mit einem H₀-Wert < +2, vorzugsweise < -3 verwendet wird.

17. Verfahren gemäß Anspruch 16, **dadurch gekennzeichnet, dass** Zeolithe, Festkörpersäuren, unedle Mischoxidkatalysatoren oder saure Ionenaustauscherharze als heterogener Katalysator verwendet werden, vorzugsweise
(i) natürliche oder synthetische silikatische Stoffe, insbesondere Mordenit, Montmorillonit und saure Zeolithe, insbesondere HZSM-5, MCM-22 und Zeolith Beta,
(ii) mit ein-, zwei- oder mehrbasigen anorganischen Säuren, insbesondere Phosphorsäure, Schwefelsäure oder sauren Salzen der anorganischen Säuren belegte Trägermaterialien, wie insbesondere oxidische oder silikatische Stoffe, insbesondere Aluminiumoxid, Titandioxid, Siliziumdioxid, Zirkondioxid oder deren Mischungen,
(iii) Oxide und Mischoxide, insbesondere Aluminiumoxide, Zinkoxid-Aluminiumoxid-Mischungen oder Heteropolysäuren, oder
(iv) (Polystyrolsulfonsäureharze, insbesondere Lewatit- oder Amberlite-Harze, oder perfluorierte Polymersulfonsäureharze, insbesondere Nafion.

18. Verfahren gemäß Anspruch 11-14, **dadurch gekennzeichnet, dass** die Dehydratisierungsreaktion b) mit Hilfe eines Biokatalysator durchgeführt wird, vorzugsweise einer Dehydratase, Hydratase oder Hydrolase.

19. Verfahren gemäß einem der Ansprüche 11 bis 18, **dadurch gekennzeichnet, dass** die Reaktion b) in Gegenwart eines Lösungsmittels durchgeführt wird, vorzugsweise
Methyl-tert.-butylether, Ethyl-tert.-butylether, Tetrahydrofuran, 1,4-Dioxan, Diglyme, Toluol, Methylisobutylketon und/oder der zur Umesterung gemäß Anspruch 1 benutzte Alkohol R'-OH .

20. Verfahren gemäß einem der Ansprüche 11 bis 19, **dadurch gekennzeichnet, dass** Glycerin aus dem Reaktionsgemisch a) durch Reaktivdestillation abgetrennt wird,
und dabei am gleichzeitig vorhandenen sauren Katalysator zu Acrolein dehydratisiert wird, oder
**dadurch gekennzeichnet, dass** Glycerin aus der Reaktionslösung durch Membrantrennung abgetrennt wird und
als Membran organische oder keramische Materialien verwendet werden, oder
**dadurch gekennzeichnet, dass** Glycerin aus der Reaktionslösung durch Phasentrennung abgetrennt wird, nachdem sich eine Fettsäurealkylester enthaltende lipophile Phase A und eine Glycerin-haltige hydrophile Phase B gebildet haben, und
die Phasentrennung durch Zugabe eines Phasentrennmittels ermöglicht oder befördert wird und bevorzugt
als Phasentrennmittel Wasser und/oder ein organisches Lösungsmittel und/oder Fettsäurealkylester verwendet werden.

21. Verfahren gemäß einem der Ansprüche 11 oder 13 bis 19, **dadurch gekennzeichnet, dass** die Reaktionen a) und b) parallel in zwei verschiedenen Flüssigphasen ablaufen, bevorzugt dabei
Reaktion a) in einer lipophilen Phase A, die Triglycerid und Fettsäurealkylester enthält, durchgeführt wird und ganz besonders bevorzugt
in der lipophilen Phase A ein dort bevorzugt löslicher Katalysator die Reaktion a) beschleunigt, oder bevorzugt dabei
Reaktion b) in einer hydrophilen Phase B, die Glycerin, Wasser und Acrolein enthält, durchgeführt wird und ganz besonders bevorzugt
in der hydrophilen Phase B ein dort bevorzugt löslicher Katalysator die Reaktion b) beschleunigt.

22. Verfahren gemäß Anspruch 21, **dadurch gekennzeichnet, dass** die Fettsäurealkylester der Formel I aus der Phase A gewonnen werden, oder
Acrolein aus der Phase B gewonnen wird.

## Claims

1. Process for preparing fatty acid alkyl esters of the general formula I: 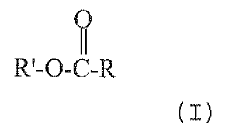 and acrolein by converting triglycerides of the general formula II: 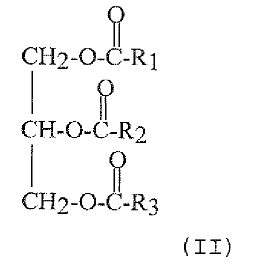 **characterized in that**
a) the triglycerides are reacted by means of alcohols R'-OH in the presence of a suitable catalyst to give the fatty acid alkyl ester and glycerol and
b) the glycerol formed is dehydrated catalytically to acrolein,
where R is R₁, R₂ and R₃, and R₁, R₂ and R₃ are all the same or some are the same or all are different and are each a straight-chain or branched and optionally mono- or polyunsaturated C₁₀-C₃₀-alkyl radical, preferably C₁₂-C₂₀-alkyl radical, and R' is a C₁-C₁₀-alkyl radical, preferably C₁-C₆-alkyl radical, a C₃-C₆-cycloalkyl radical, preferably C₆-cycloalkyl radical, and wherein reactions a) and b) are performed simultaneously in one step.

2. Process according to Claim 1, **characterized in that**
R₁, R₂ and R₃ = C₁₂-C₁₈-alkyl radical, and/or
R' = methyl or ethyl.

3. Process according to either of Claims 1 and 2, **characterized in that** a homogeneous or heterogeneous acidic catalyst and/or salts of mineral acids or a biocatalyst are used for reactions a) and b).

4. Process according to Claim 3, **characterized in that** a homogeneous catalyst with a pKa of < 7 is used, preferably sulphuric acid, phosphoric acid, toluenesulphonic acid or methanesulphonic acid.

5. Process according to Claim 3, **characterized in that** potassium sulphate, sodium sulphate or caesium sulphate, potassium hydrogensulphate, sodium hydrogensulphate or caesium hydrogensulphate, or mixtures of the hydrogensulphates and sulphates mentioned, lithium phosphate, iron phosphate, zinc sulphate, optionally in the presence of a solvent, are used as a catalyst.

6. Process according to Claim 3, **characterized in that** a heterogeneous catalyst with an H₀ value of < +2 is used, preferably with an H₀ value of < -3, more preferably the zeolites, solid acids, mixed base metal oxide catalysts or acidic ion exchange resins as the heterogeneous catalyst.

7. Process according to Claim 6, **characterized in that**
(i) natural or synthetic silicatic substances, especially mordenite, montmorillonite and acidic zeolites, especially HZSM-5, MCM-22 and zeolite beta,
(ii) support materials, especially oxidic or silicatic substances, preferably aluminium oxide, titanium dioxide, silicon dioxide, zirconium dioxide or mixtures thereof, coated with mono-, di- or polybasic inorganic acids, especially phosphoric acid, sulphuric acid or acidic salts of inorganic acids,
(iii) oxides and mixed oxides, especially aluminium oxides, zinc oxide-aluminium oxide mixtures or heteropolyacids, or
(iv) polystyrenesulphonic acid resins, especially Lewatit or Amberlite resins or perfluorinated polymeric sulphonic acid resins, especially Nafion, are used.

8. Process according to Claim 3, **characterized in that** lipases, esterases, hydratases and/or hydrolases are used as a biocatalyst.

9. Process according to one of Claims 1 to 8, **characterized in that** the reaction is performed in the presence of a solvent, preferably methyl tert-butyl ether, ethyl tert-butyl ether, tetrahydrofuran, 1,4-dioxane, diglyme, toluene, methyl isobutyl ketone and/or the alcohol R'-OH utilized for the transesterification according to Claim 1.

10. Process according to one of Claims 1 to 9, **characterized in that** the acrolein formed in reaction b) is removed from the reaction mixture alone or optionally together with at least a portion of the solvent, preferably by stripping, distillation, extraction, phase separation or use of membranes, and the acrolein is most preferably removed from the reaction mixture by distillation actually during the reaction.

11. Process for preparing fatty acid alkyl esters of the general formula I: 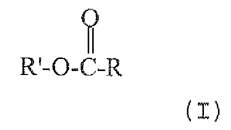 and acrolein by converting triglycerides of the general formula II: 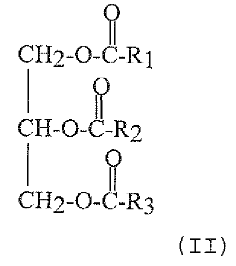 **characterized in that**
a) the triglycerides are reacted by means of alcohols R'-OH in the presence of a suitable catalyst to give the fatty acid alkyl ester and glycerol and
b) the glycerol formed is dehydrated catalytically to acrolein,
where R is R₁, R₂ and R₃, and R₁, R₂ and R₃ are all the same or some are the same or all are different and are each a straight-chain or branched and optionally mono- or polyunsaturated C₁₀-C₃₀-alkyl radical, preferably C₁₂-C₂₀-alkyl radical, and R' is a C₁-C₁₀-alkyl radical, preferably C₁-C₆-alkyl radical, a C₃-C₆-cycloalkyl radical, preferably C₆-cycloalkyl radical, and wherein reactions a) and b) are performed simultaneously in spatially separate steps and the glycerol formed as the primary product from the reaction mixture of reaction a) is removed completely or partly by membrane separation, by phase separation after a lipophilic phase A comprising fatty acid alkyl esters and a glycerol-containing hydrophilic phase B have formed, or by reactive distillation, and is dehydrated catalytically to acrolein in reaction b)

12. Process according to Claim 11, **characterized in that** reaction a) is performed with the aid of an alkaline catalyst for which the pKa of the corresponding acid is > 13, **in that** preferably the alkaline catalysts used are alkali metal alkoxides of the alcohol R'-OH used for the transesterification or alkali metal methoxides or ethoxides, more preferably sodium methoxide.

13. Process according to Claim 11, **characterized in that** reaction a) is performed with the aid of a homogeneous or heterogeneous acidic catalyst, preferably a homogeneous acidic catalyst with a pKa of < 7 is used, or **characterized in that** a heterogeneous catalyst with an H₀ of < +2, preferably < -3, is used.

14. Process according to Claim 11, **characterized in that** reaction a) is performed with the aid of a biocatalyst, preferably a lipase, esterase, hydratase or hydrolase.

15. Process according to Claims 11-14, **characterized in that** a homogeneous or heterogeneous acidic catalyst is used for the dehydration reaction b), preferably a homogeneous catalyst with a pKa of < 7, most preferably sulphuric acid, phosphoric acid, toluenesulphonic acid or methanesulphonic acid.

16. Process according to Claims 11-14, **characterized in that** salts of mineral acids are used as catalysts for the dehydration reaction b), preferably potassium sulphate, sodium sulphate or caesium sulphate, potassium hydrogensulphate, sodium hydrogensulphate or caesium hydrogensulphate, or mixtures of the hydrogensulphates and sulphates mentioned, lithium phosphate, iron phosphate, zinc sulphate, optionally in the presence of a solvent, or **in that** a heterogeneous catalyst with an H₀ of < +2, preferably < -3, is used for the dehydration reaction b).

17. Process according to Claim 16, **characterized in that** the zeolites, solid acids, mixed base metal oxide catalysts or acidic ion exchange resins are used as the heterogeneous catalyst, preferably
(i) natural or synthetic silicatic substances, especially mordenite, montmorillonite and acidic zeolites, especially HZSM-5, MCM-22 and zeolite beta,
(ii) support materials, especially oxidic or silicatic substances, preferably aluminium oxide, titanium dioxide, silicon dioxide, zirconium dioxide or mixtures thereof, coated with mono-, di- or polybasic inorganic acids, especially phosphoric acid, sulphuric acid or acidic salts of inorganic acids,
(iii) oxides and mixed oxides, especially aluminium oxides, zinc oxide-aluminium oxide mixtures or heteropolyacids, or
(iv) polystyrenesulphonic acid resins, especially Lewatit or Amberlite resins or perfluorinated polymeric sulphonic acid resins, especially Nafion.

18. Process according to Claims 11-14, **characterized in that** the dehydration reaction b) is performed with the aid of a biocatalyst, preferably a dehydratase, hydratase or hydrolase.

19. Process according to one of Claims 11 to 18, **characterized in that** reaction b) is performed in the presence of a solvent, preferably methyl tert-butyl ether, ethyl tert-butyl ether, tetrahydrofuran, 1,4-dioxane, diglyme, toluene, methyl isobutyl ketone and/or the alcohol R'-OH utilized for the transesterification according to Claim 1.

20. Process according to one of Claims 11 to 19, **characterized in that** glycerol is removed from the reaction mixture a) by reactive distillation and, at the same time, is dehydrated to acrolein over the acidic catalyst present simultaneously, or **characterized in that** glycerol is removed from the reaction solution by membrane separation and the membranes used are organic or ceramic materials, or **characterized in that** glycerol is removed from the reaction solution by phase separation after a lipophilic phase A comprising fatty acid alkyl esters and a glycerol-containing hydrophilic phase B have formed, and the phase separation is enabled or promoted by addition of a phase separation agent and preferably the phase separation agents used are water and/or an organic solvent and/or fatty acid alkyl esters.

21. Process according to one of Claims 11 or 13 to 19, **characterized in that** reactions a) and b) proceed in parallel in two different liquid phases, reaction a) preferably being performed in a lipophilic phase A which comprises triglyceride and fatty acid alkyl esters and a catalyst which is preferably soluble in the lipophilic phase A most preferably accelerating the reaction a) therein, or reaction b) preferably being performed in a hydrophilic phase B which comprises glycerol, water and acrolein and a catalyst which is preferably soluble in the hydrophilic phase B most preferably accelerating the reaction b) therein.

22. Process according to Claim 21, **characterized in that** the fatty acid alkyl esters of the formula I are obtained from the phase A, or acrolein is obtained from the phase B.

## Revendications

1. Procédé pour la préparation d'esters alkyliques d'acides gras de formule générale I : 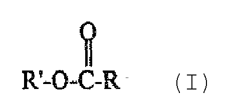 et d'acroléine, par conversion de triglycérides de formule générale II : 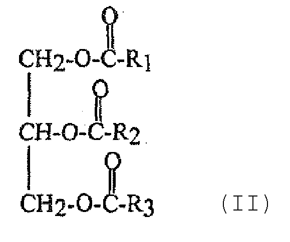 **caractérisé en ce que**
a) on convertit les triglycérides, au moyen d'alcools R'-OH, en présence d'un catalyseur approprié, en l'ester alkylique d'acide gras et glycérol et
b) le glycérol résultant est déshydraté par voie catalytique en acroléine,
R représentant R₁, R₂ et R₃ et R₁, R₂ et R₃ étant tous identiques ou en partie identiques ou
tous différents et représentant chacun un radical alkyle en C₁₀-C₃₀, de préférence un radical alkyle en C₁₂-C₂₀, à chaîne droite ou ramifiée et éventuellement une ou plusieurs fois insaturé, et R' représentant un radical alkyle en C₁-C₁₀, de préférence un radical alkyle en C₁-C₆, un radical cycloalkyle en C₃-C₆, de préférence un radical cycloalkyle en C₆, et les réactions a) et b) étant effectuées simultanément en une étape.

2. Procédé selon la revendication 1, **caractérisé en ce que**
R₁, R₂ et R₃ est un radical alkyle en C₁₂-C₁₈ et/ou
R' est le groupe méthyle ou éthyle.

3. Procédé selon l'une quelconque des revendications 1 et 2, **caractérisé en ce que** pour les réactions a) et b) on utilise un catalyseur acide homogène ou hétérogène et/ou des sels d'acides minéraux ou un biocatalyseur.

4. Procédé selon la revendication 3, **caractérisé en ce qu'**on utilise un catalyseur homogène ayant une valeur pKₐ < 7, de préférence
l'acide sulfurique, l'acide phosphorique, l'acide toluènesulfonique ou l'acide méthanesulfonique.

5. Procédé selon la revendication 3, **caractérisé en ce qu'**on utilise comme catalyseur le sulfate de potassium, de sodium ou de césium, l'hydrogénosulfate de potassium, de sodium ou de césium ou des mélanges des sulfates et hydrogénosulfates nommés, le phosphate de lithium, le phosphate de fer, le sulfate de zinc, éventuellement en présence d'un solvant.

6. Procédé selon la revendication 3, **caractérisé en ce qu'**on utilise un catalyseur hétérogène ayant un indice H₀ < +2, de préférence ayant un indice H₀ < -3,
de façon particulièrement préférée des zéolithes, des acides solides, des catalyseurs de type oxyde mixte commun ou des résines échangeuses d'ions acides en tant que catalyseur hétérogène.

7. Procédé selon la revendication 6, **caractérisé en ce qu'**on utilise
(i) des substances de type silicate naturelles ou synthétiques, en particulier la mordénite, la montmorillonite et des zéolithes acides, en particulier HZSM-5, MCM-22 et la zéolithe bêta,
(ii) des matières de support, comme en particulier des substances de type oxyde ou de type silicate, de préférence l'oxyde d'aluminium, le dioxyde de titane, le dioxyde de silicium, le dioxyde de zirconium ou des mélanges de ceux-ci, chargées avec des acides inorganiques mono-, di- ou polybasiques, en particulier l'acide phosphorique, l'acide sulfurique ou des sels acides d'acides inorganiques,
(iii) des oxydes et oxydes mixtes, en particulier des oxydes d'aluminium, des mélanges d'oxyde de zinc-oxyde d'aluminium ou des hétéropolyacides, ou
(iv) des résines polystyrène sulfonées, en particulier les résines Lewatit ou Amberlite ou des résines polymères sulfonées perfluorées, en particulier le Nafion.

8. Procédé selon la revendication 3, **caractérisé en ce qu'**on utilise comme biocatalyseur des lipases, estérases, hydratases et/ou hydrolases.

9. Procédé selon l'une quelconque des revendications 1 à 8, **caractérisé en ce que** la réaction est effectuée en présence d'un solvant, de préférence
l'oxyde de méthyle et de tert-butyle, l'oxyde d'éthyle et de tert-butyle, le tétrahydrofurane, le 1,4-dioxane, le diglyme, le toluène, la méthylisobutylcétone et/ou l'alcool R'-OH utilisé dans la transestérification selon la revendication 1.

10. Procédé selon l'une quelconque des revendications 1 à 9, **caractérisé en ce que** l'acroléine formée dans la réaction b) est séparée du mélange réactionnel seule ou éventuellement conjointement avec au moins une partie du solvant, de préférence
par entraînement, distillation, extraction, séparation de phases ou utilisation de membranes, de façon tout particulièrement préférée
on sépare l'acroléine du mélange réactionnel par distillation déjà pendant la réaction.

11. Procédé pour la préparation d'esters alkyliques d'acides gras de formule générale I : 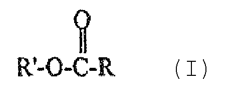 et d'acroléine, par conversion de triglycérides de formule générale II : 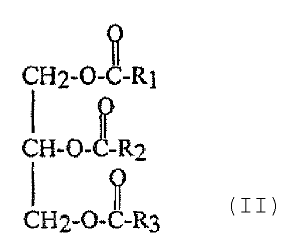 **caractérisé en ce que**
a) on convertit les triglycérides, au moyen d'alcools R'-OH, en présence d'un catalyseur approprié, en l'ester alkylique d'acide gras et glycérol et
b) le glycérol résultant est déshydraté par voie catalytique en acroléine,
R représentant R₁, R₂ et R₃ et R₁, R₂ et R₃ étant tous identiques ou en partie identiques ou
tous différents et représentant chacun un radical alkyle en C₁₀-C₃₀, de préférence un radical alkyle en C₁₂-C₂₀, à chaîne droite ou ramifiée et éventuellement une ou plusieurs fois insaturé, et R' représentant un radical alkyle en C₁-C₁₀, de préférence un radical alkyle en C₁-C₆, un radical cycloalkyle en C₃-C₆, de préférence un radical cycloalkyle en C₆, et les réactions a) et b) étant effectuées simultanément dans des étapes séparées dans l'espace et le glycérol formé en premier lieu est séparé en totalité ou en partie du mélange réactionnel de la réaction a) par séparation sur membrane, par séparation de phases après formation d'une phase lipophile A contenant des esters alkyliques d'acides gras et d'une phase hydrophile B contenant du glycérol ou par distillation réactive, et est déshydraté par voie catalytique en acroléine dans la réaction b).

12. Procédé selon la revendication 11, **caractérisé en ce que** la réaction a) est effectuée à l'aide d'un catalyseur alcalin, pour lequel l'indice pKa de l'acide correspondant est > 13,
**en ce que** comme catalyseur alcalin on utilise de préférence des alcoolates de métaux alcalins de l'alcool R'OH utilisé pour la transestérification ou des méthylates ou éthylates de métaux alcalins, de façon particulièrement préférée le méthylate de sodium.

13. Procédé selon la revendication 11, **caractérisé en ce qu'**on effectue la réaction a) à l'aide d'un catalyseur acide homogène ou hétérogène,
on utilise de préférence un catalyseur acide homogène ayant un pKa < 7, ou
**caractérisé en ce qu'**on utilise un catalyseur hétérogène ayant un indice H₀ < +2, de préférence < -3.

14. Procédé selon la revendication 11, **caractérisé en ce qu'**on effectue la réaction a) à l'aide d'un biocatalyseur, de préférence
une lipase, estérase, hydratase ou hydrolase.

15. Procédé selon l'une quelconque des revendications 11 à 14, **caractérisé en ce que** pour la réaction de déshydratation b) on utilise un catalyseur acide homogène ou hétérogène,
de préférence un catalyseur homogène ayant un pKa < 7, de façon tout particulièrement préférée
l'acide sulfurique, l'acide phosphorique, l'acide toluènesulfonique ou l'acide méthanesulfonique.

16. Procédé selon l'une quelconque des revendications 11 à 14, **caractérisé en ce que** pour la réaction de déshydratation b) on utilise comme catalyseurs des sels d'acides minéraux, de préférence
le sulfate de potassium, de sodium ou de césium, l'hydrogénosulfate de potassium, de sodium ou de césium ou des mélanges des sulfates et hydrogénosulfates nommés précédemment, le phosphate de lithium, le phosphate de fer, le sulfate de zinc, éventuellement en présence d'un solvant, ou
**en ce que** pour la réaction de déshydratation b) on utilise un catalyseur hétérogène ayant un indice H₀ < +2, de préférence < -3.

17. Procédé selon la revendication 16, **caractérisé en ce qu'**on utilise comme catalyseur hétérogène des zéolithes, des acides solides, des catalyseurs de type oxyde mixte commun ou des résines échangeuses d'ions acides, de préférence
(i) des substances de type silicate naturelles ou synthétiques, en particulier la mordénite, la montmorillonite et des zéolithes acides, en particulier HZSM-5, MCM-22 et la zéolithe bêta,
(ii) des matières de support, comme en particulier des substances de type oxyde ou de type silicate, en particulier l'oxyde d'aluminium, le dioxyde de titane, le dioxyde de silicium, le dioxyde de zirconium ou des mélanges de ceux-ci, chargées avec des acides inorganiques mono-, di- ou polybasiques, en particulier l'acide phosphorique, l'acide sulfurique ou des sels acides des acides inorganiques,
(iii) des oxydes et oxydes mixtes, en particulier des oxydes d'aluminium, des mélanges d'oxyde de zinc-oxyde d'aluminium ou des hétéropolyacides, ou
(iv) des résines polystyrène sulfonées, en particulier les résines Lewatit ou Amberlite, ou des résines polymères sulfonées perfluorées, en particulier le Nafion.

18. Procédé selon l'une quelconque des revendications 11 à 14, **caractérisé en ce que** la réaction de déshydratation b) est effectuée à l'aide d'un biocatalyseur, de préférence
une déshydratase, hydratase ou hydrolase.

19. Procédé selon l'une quelconque des revendications 11 à 18, **caractérisé en ce que** la réaction b) est effectuée en présence d'un solvant, de préférence
l'oxyde de méthyle et de tert-butyle, l'oxyde d'éthyle et de tert-butyle, le tétrahydrofurane, le 1,4-dioxane, le diglyme, le toluène, la méthylisobutylcétone et/ou l'alcool R'-OH utilisé dans la transestérification selon la revendication 1.

20. Procédé selon l'une quelconque des revendications 11 à 19, **caractérisé en ce que** le glycérol est séparé du mélange réactionnel a) par distillation réactive,
et en outre est déshydraté en acroléine sur le catalyseur acide présent simultanément, ou
**caractérisé en ce que** le glycérol est séparé de la solution réactionnelle par séparation sur membrane et
comme membrane on utilise des matériaux céramiques ou organiques, ou
**caractérisé en ce que** le glycérol est séparé de la solution réactionnelle par séparation de phases, après formation d'une phase lipophile A contenant des esters alkyliques d'acides gras et une phase hydrophile B contenant du glycérol et
la séparation de phases est permise ou facilitée par addition d'un agent de séparation de phases et de préférence
on utilise comme agent de séparation de phases l'eau et/ou un solvant organique et/ou un ester alkylique d'acide gras.

21. Procédé selon l'une quelconque des revendications 11 ou 13 à 19, **caractérisé en ce que** les réactions a) et b) se déroulent parallèlement dans deux phases liquides différentes, de préférence
en ce cas la réaction a) est effectuée dans une phase lipophile A, qui contient un triglycéride et des esters alkyliques d'acides gras, et de façon tout particulièrement préférée
dans la phase lipophile A un catalyseur de préférence soluble dans celle-ci accélère la réaction a), ou de préférence en ce cas
la réaction b) est effectuée dans une phase hydrophile B, qui contient du glycérol, de l'eau et de l'acroléine et de façon tout particulièrement préférée
dans la phase hydrophile B un catalyseur de préférence soluble dans celle-ci accélère la réaction b).

22. Procédé selon la revendication 21, **caractérisé en ce que** les esters alkyliques d'acides gras de formule I sont obtenus à partir de la phase A, ou l'acroléine est obtenue à partir de la phase B.
